Europäisches Patentamt

European Patent Office

Office européen des brevets

(11) Publication number: **0 115 931**
**B1**

# EUROPEAN PATENT SPECIFICATION

(45) Date of publication of patent specification: **18.05.88**

(51) Int. Cl.⁴: **A 61 M 5/315,** A 61 M 5/31

(21) Application number: **84300393.0**

(22) Date of filing: **23.01.84**

(54) **Syringe.**

(30) Priority: **23.01.83 IL 67736**
**25.11.83 IL 70331**

(43) Date of publication of application:
**15.08.84 Bulletin 84/33**

(45) Publication of the grant of the patent:
**18.05.88 Bulletin 88/20**

(84) Designated Contracting States:
**AT BE CH DE FR GB IT LI NL SE**

(56) References cited:
**EP-A-0 058 536**
**DE-C- 167 058**
**DE-C- 516 791**
**FR-A-1 216 753**
**GB-A- 510 484**
**US-A-1 707 880**
**US-A-2 515 956**
**US-A-2 561 273**
**US-A-2 646 042**
**US-A-3 327 904**
**US-A-3 815 785**
**US-A-4 261 359**

(73) Proprietor: **Porat, Amir**
**4, Hirschenberg St.**
**Tel Aviv (IL)**
(73) Proprietor: **Porat, Michael**
**4, Hirschenberg St**
**Tel Aviv (IL)**
(73) Proprietor: **Lachish, Doron**
**11, Ester Hamalka Street**
**Tel Aviv (IL)**

(72) Inventor: **Porat, Amir**
**4, Hirschenberg St.**
**Tel Aviv (IL)**
Inventor: **Porat, Michael**
**4, Hirschenberg St**
**Tel Aviv (IL)**
Inventor: **Lachish, Doron**
**11, Ester Hamalka Street**
**Tel Aviv (IL)**

(74) Representative: **Needle, Jacqueline et al**
**PAGE, WHITE & FARRER 5 Plough Place New**
**Fetter Lane**
**London EC4A 1HY (GB)**

Courier Press, Leamington Spa, England.

## Description

The present invention relates to a syringe in which a predetermined quantity of medication can be stored to be dispensed in predetermined doses as required.

In medicine, for example for diabetic patients, or in dentistry, for example for anaesthetic purposes, individual disposable syringes are provided, each being used for one dose of the injection required. It is clear that this is a wasteful and costly procedure, particularly since in most cases the same quantity of medication is required for each injection. Furthermore, it is often desirable that the physician fills a syringe with the quantity of medication sufficient for the entire course of treatment and adjusts the syringe so that the patient, upon each actuation of the syringe will always inject only the required dose.

US—A—2 646 042 describes a syringe for storing medication and dispensing dosed quantities thereof comprising:

a housing;

a storage chamber within said housing and defining an outlet opening therein;

a dose chamber in said housing in direct communication with said storage chamber;

first piston means partially within said housing for initially introducing a fluid into said storage chamber through said outlet opening and subsequently for introducing by pressure a predetermined volume of the fluid into said dose chamber; and

second piston means partially within said first piston means for expelling the volume of fluid from the dose chamber;

said first piston means being in direct communication with the storage chamber and operable to vary the volume thereof for a selective increase in the volume to permit the inflow of fluid therein, and for a selective reduction in the volume for generating an increase of pressure therein;

said second piston means being in direct communication with said dose chamber and operable to vary the volume thereof and mounted for outward movement in response to an increase in the volume of fluid in said dose chamber and for manual inward movement;

means associated with said second piston means for indicating the volume of fluid in said dose chamber;

said first and second piston means being coaxial, said first piston means having an inner end and an outer end, said second piston means extending axially through said first piston means with the inner end of the second piston means in said dose chamber and the outer end of the second piston means at the outer end of said first piston means;

means for preventing relative movement between said first piston means and said housing when said second piston means is moved to expel fluid from the dose chamber;

sealing means mounted adjacent the outlet opening of said storage chamber and selectively openable to allow fluid movement therethrough.

This known syringe enables a predetermined quantity of a medicament to be introduced therein at any given time but requires constant control by the user.

It is an object of this invention to provide a syringe which is adapted to store a quantity of medication for multiple injections and with which predetermined doses of this medication can be expelled.

The present invention is a syringe characterised in that said means for preventing relative movement comprises engaged screw thread means on and between said first piston means and said housing arranged such that rotational movement of said first piston means relative to said housing will effect a longitudinal movement of said first piston means relative to said housing and the storage chamber therein;

said means for indicating the volume of fluid in said dose chamber comprises a tubular handle received over said second piston means and having calibrations thereon; means on said handle to adjustably limit outward movement of said second piston means; and

said dose chamber is defined by an elongate sleeve opening through the inner end of said first piston means, said second piston means being received in said sleeve, said means to adjustably limit outward movement of said second piston means comprising a nut adjustably mounted on said sleeve, and engaged for rotation by the interior of said tubular handle, said handle being longitudinally slidable over said nut, and abutment means on said handle engageable with the nut, as adjusted, to limit outward movement of said handle and said second piston means.

Embodiments of the present invention will hereinafter be described, by way of example, with reference to the accompanying drawings in which:

Figure 1, shows a longitudinal schematic section of an embodiment of a syringe of the invention, and

Figure 2 shows the radial calibration provided on the syringe shown in Figure 1.

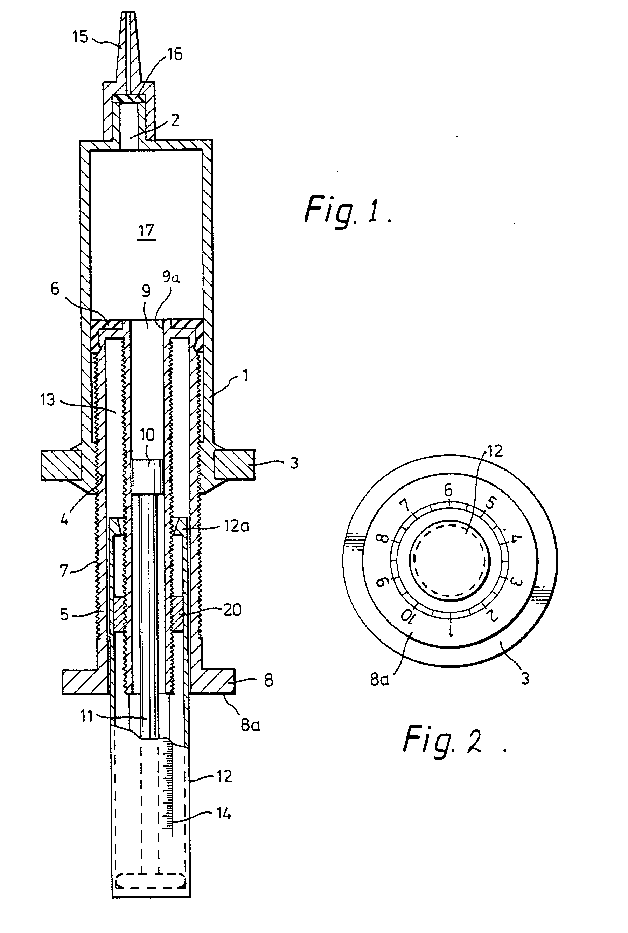

The syringe shown in Figure 1 has an outer syringe housing 1 provided with an outlet opening 2 at the closed end and a circumferential flange 3 at the open end, a short internal thread 4 also being provided at this open end. A cylindrical casing 5, having an external thread 7 along its length, is rotatably mounted within housing 1, the inner end 6 of the casing 5 constituting a piston. At its end opposite to the inner end 6, the casing 5 is provided with a circumferential flange 8. The casing 5 has an axially extending cylindrical sleeve 9a which merges with end 6 and in which a piston 10 is longitudinally movable by its rod 11. This rod 11 is enclosed by a tubular handle 12 which extends into an annular well 13 in casing 5 surrounding sleeve 9a. The tube 12 is arranged to engage the rod 11 such that it can be moved therewith. The tube 12 is linearly calibrated at 14 as indicated.

It is a particular feature of the syringe illustrated that it can be used to repeatedly administer a fixed dose. For this purpose the tubular handle 12 has a hexagonal internal cross-section, a hexagonal nut 20 being housed in its inner end and being held therein by an internal flange 12a. Thus, the handle 12 can freely move axially and when rotated will force the nut 20 to rotate. The tubular wall of sleeve 9a is provided with an external thread along its length adapted to be screwingly engaged by the nut 20. The circumferential flange 8 is provided with radial calibrations 8a, ten being shown here, the relationship between calibration 14 and calibration 8a being similar to that used in a micrometer.

The syringe works as follows:

A two way hypodermic needle (not shown) which is double-sided, is attached in the conventional manner to a cap 15 which covers the end of outlet opening 2 with the interposition of sealing means in the form of an elastic washer 16, the needle penetrating this washer 16. A storage chamber 17 is defined between the inner end 6 of the casing 5 and the closed end of the housing 1.

Medicament is introduced into this storage chamber 17 through the outlet opening 2 via the washer 16, and the double-sided needle, by retracting the first piston 6. The piston 6 is retracted by rotating the casing 5 relative to the housing 1 by way of the screw threads 4 and 7. When the storage chamber 17 has received the desired amount of medicament, normally an amount equivalent to several doses, the outlet opening 2 is closed by removal of the needle, thereby providing a sealed container.

When a dose is to be administered, the handle 12 is rotated to thus rotate the nut 20 until the desired calibration 14 appears against the flange 8. Now the casing 5 is rotated, the outlet 2 remaining closed, with the internal pressure generated forcing the medicament into a dose chamber 9. This movement of the medicament into the dose chamber 9 will move the piston 10 together with the handle 12 in an outward direction, the piston 10 being stopped in its movement when flange 12a meets the nut 20 such that a predetermined dose will be filled in the chamber 9. In order to dispense said predetermined dose a second hypodermic needle, which is a finer one, is attached and penetrates washer 16. An amount of medicament equal to the predetermined dose within the dose chamber 9 is then injected. This is effected by pushing handle 12 inwardly to drive the piston 10 inwardly. This inward driving of the second piston 10 moves the dose amount of medicament from the chamber 9 to the chamber 17, at the same time discharging an equal amount of medicament through the outlet opening 2. The provision of screw threads between the first piston 6 and the housing 1 prevents movement of the first piston 6 during the outward and inward movement of the second piston 10. A stop (not shown) prevents further movement after the end of the piston 10 has reached the end 6 of the casing 5.

After the needle has been removed, the piston 10 will remain in its position until a further injection is made. At this time the casing 5 will again be screwed into the housing 1 forcing piston 10 outwardly thereby filling dose chamber 9 with the required dose. The next dosage will now be exactly that of the first one. Since the calibration 14 may be difficult to read, calibration 8a will show a more accurate reading.

The profile of the internal wall of the handle 12 may have any other shape than that described, e.g. square or any other geometric shape, or a circular nut may be provided with diametrically opposed lugs which engage in diametrically opposed elongated slits in the wall of the handle 12.

The calibrations may be raised, so that the syringe can be used by the blind.

**Claims**

1. A syringe for storing medication and dispensing dosed quantities thereof comprising:
a housing (1);
a storage chamber (17) within said housing and defining an outlet opening therein;
a dose chamber (9) in said housing in direct communication with said storage chamber (17);
first piston means (6) partially within said housing for initially introducing a fluid into said storage chamber (17) through said outlet opening and subsequently for introducing by pressure a predetermined volume of the fluid into said dose chamber (9); and
second piston means (10) partially within said first piston means for expelling the volume of fluid from the dose chamber (9);
said first piston means (6) being in direct communication with the storage chamber (17) and operable to vary the volume thereof for a selective increase in the volume to permit the inflow of fluid therein, and for a selective reduction in the volume for generating an increase of pressure therein;
said second piston means (10) being in direct communication with said dose chamber (9) and operable to vary the volume thereof and mounted for outward movement in response to an increase in the volume of fluid in said dose chamber and for manual inward movement;
means (14) associated with said second piston means (10) for indicating the volume of fluid in said dose chamber (9); said first and second piston means (6, 10) being coaxial, said first piston means (6) having an inner end and an outer end, said second piston means (10) extending axially through said first piston means with the inner end of the second piston means in said dose chamber (9) and the outer end of the second piston means at the outer end of said first piston means;

means (4, 7) for preventing relative movement between said first piston means (6) and said housing (1) when said second piston means (10) is moved to expel fluid from the dose chamber (9);

sealing means (16) mounted adjacent the outlet opening of said storage chamber (17) and selectively openable to allow fluid movement therethrough;

characterised in that said means (4, 7) for preventing relative movement comprises engaged screw thread means (4, 7) on and between said first piston means (6) and said housing (1) arranged such that rotational movement of said first piston means (6) relative to said housing (1) will effect a longitudinal movement of said first piston means (6) relative to said housing and the storage chamber therein;

said means for indicating the volume of fluid in said dose chamber (9) comprises a tubular handle (12) received over said second piston means (10) and having calibrations (14) thereon;

means (18; 20) on said handle (12) to adjustably limit outward movement of said second piston means (10); and

said dose chamber (9) is defined by an elongate sleeve (9a) opening through the inner end of said first piston means (6), said second piston means (10) being received in said sleeve, said means to adjustably limit outward movement of said second piston means (10) comprising a nut (20) adjustably mounted on said sleeve (9a), and engaged for rotation by the interior of said tubular handle, said handle (12) being longitudinally slidable over said nut, and abutment means (12a) on said handle engageable with the nut, as adjusted, to limit outward movement of said handle (12) and said second piston means (10).

2. A syringe as claimed in Claim 1, wherein said sealing means comprises an elastic washer (16) which can be penetrated by a hypodermic needle and which seals itself once the needle is withdrawn.

3. A syringe as claimed in Claim 1 or Claim 2, wherein radial calibrations (8a) are provided on a flange (8) on and surrounding said first piston means (6).

**Patentansprüche**

1. Eine Spritze zum Speichern von Arzneimittel und zur Verabreichung dosierter Mengen davon mit:

einem Gehäuse (1);

einer Speicherkammer (17) innerhalb des besagten Gehäuses, die eine darin befindliche Ausgangsöffnung abgrenzt;

einer Dosierkammer (9) innerhalb des besagten Gehäuses in unmittelbarer Verbindung mit der besagten Speicherkammer (17);

einer ersten Kolbeneinheit (6), die sich zum Teil innerhalb des besagten Gehäuses befindet, für anfängliche Einführung einer Flüssigkeit in die besagte Speicherkammer (17) durch die besagte Ausgangsöffnung hindurch, und danach zwecks Einführung durch Druck einer vorbestimmten Menge der Flüssigkeit in die besagte Dosierkammer (9);

wobei die besagte erste Kolbeneinheit (6) mit der Speicherkammer (17) in unmittelbarer Verbindung ist und so betätigt werden kann, daß deren Volumen geändert wird, um das Volumen selektiv zu vergrößern, so daß die Flüssigkeit in die besagte Kammer einfließen kann, und das Volumen selektiv verringert, um eine Erhöhung des Druckes innerhalb der Kammer zu bewirken;

wobei die besagte zweite Kolbeneinheit (10) in unmittelbarer Verbindung mit der besagten Dosierkammer (9) steht und so betätigt werden kann, daß deren Volumen geändert wird, und so angeordnet ist, daß sie Bewegung nach außen gestattet, wenn die Flüssigkeitsmenge in der besagten Dosierkammer zunimmt — sowie, von Hand, Bewegung nach innen; ein Mittel (14), das mit der besagten zweiten Kolbeneinheit (10) in Verbindung steht und dazu dient, die Flüssigkeitsmenge in der besagten Dosierkammer (9) anzuzeigen;

wobei die besagten ersten und zweiten Kolbeneinheiten (6, 10) koaxial sind, die besagte erste Kolbeneinheit (6) ein inneres Ende und ein äußeres Ende aufweist und die besagte zweite Kolbeneinheit (10) sich axial durch die besagte erste Kolbeneinheit hindurch erstreckt, wobei das innere Ende der zweiten Kolbeneinheit in der besagten Dosierkammer (9) ist und das äußere Ende der zweiten Kolbeneinheit am äußeren Ende der besagten ersten Kolbeneinheit;

Mittel (4, 7) zur Verhütung relativer Bewegung zwischen der besagten ersten Kolbeneinheit (6) und dem besagten Gehäuse (1), wenn die besagte zweite Kolbeneinheit (10) bewegt wird, um Flüssigkeit aus der Dosierkammer (9) auszustoßen;

einer Abdichtvorrichtung (16) anschließend an der Ausgangsöffnung der besagten Speicherkammer (17) und für selektives Öffnen, um Durchgang der Flüssigkeit zu gestatten, geeignet ist;

dadurch gekennzeichnet, daß die besagten Mittel (4, 7) zum Verhüten relativer Bewegung in Eingriff befindliche Schraubgewindemittel (4, 7) an und zwischen der besagten ersten Kolbeneinheit (6) und dem besagten Gehäuse (1) umfassen, wobei diese so angeordnet sind, daß Drehbewegung der besagten ersten Kolbeneinheit (6) im Verhältnis zu dem besagten Gehäuse (1) eine Längsbewegung der besagten ersten Kolbeneinheit (6) im Verhältnis zu dem besagten Gehäuse und der darin befindlichen Speicherkammer bewirkt;

das besagte Mittel zur Anzeige der Flüssigkeitsmenge in der besagten Dosierkammer (9) einen rohrförmigen Griff (12) umfaßt, der sich über der besagten zweiten Kolbeneinheit (10) befindet und mit einer darauf befindlichen Teilung (14) versehen ist; an dem besagten Griff (12) Mittel (18, 20) vorgesehen sind, die zu verstellbarer Begrenzung der Außenbewegung der besagten zweiten Kolbeneinheit (10) dienen; und besagte Dosierkammer (9) durch eine verlängerte Muffe (9a) abgegrenzt ist, die durch das innere Ende der besagten ersten Kolbeneinheit (6) mündet, wobei die besagte zweite Kolbeneinheit (10) in der besagten Muffe sitzt, die besagten Mittel zu verstellbarer Begrenzung der Bewegung nach außen der besagten zweiten Kolbeneinheit (10) eine Mutter (20) umfassen, die einstellbar an der besagten Muffe (9a) angeordnet und zwecks Drehung durch die Innenseite des besagten rohrförmigen Griffs in Eingriff ist, der besagte Griff (12) der Länge nach über die besagte Mutter geschoben werden kann, und eine Anschlageinheit (12a) an dem besagten Griff mit der Mutter im eingestellten Zustand zum Eingriff gebracht werden kann, um die Bewegung nach außen des besagten Griffes (12) der besagten zweiten Kolbeneinheit (10) zu begrenzen.

2. Eine Spritze im Einklang mit Anspruch 1, bei der die besagte Abdichtvorrichtung eine elastische Unterlegscheibe (16) umfaß, die durch eine hypodermische Nadel durchdrungen werden kann und sich selbst abdichtet, wenn die Nadel herausgezogen wird.

3. Eine Spritze im Einklang mit Anspruch 1 oder Anspruch 2, bei der an einem Flansch (8) an und rings um die erste Kolbeneinheit (6) eine radiale Teilung (8a) vorgesehen ist.

**Revendications**

1. Une seringue pour contenir des médicaments et préparer des dosages de cela comprenant:
un corps (1);
un réservoir de contenance (17) en dedans dudit corps et définissant un ajutage de passage en cela;
une chambre de dosage (9) dans ledit corps en communication directe avec ledit réservoir (17);
système à piston primaire (6) partiellement en dedans dudit corps pour l'introduction initiale d'un fluide dans ledit réservoir (17) par ledit ajutage de passage et subséquemment pour introduire sous pression un volume prédéterminé de fluide dans ladite chambre de dosage (9); et
système à piston secondaire (10) partiellement en dedans dudit système à piston primaire pour éjecter le volume de fluide depuis la chambre de dosage (9);
ledit système à piston primaire (6) étant en communication directe avec le réservoir (17) et utilisable pour faire varier le volume de cela pour un accroissement sélectif du volume pour permettre l'admission du fluide en cela, et pour une réduction sélective en volume pour engendrer une hausse de pression en cela;
ledit système à piston secondaire (10) étant en communication directe avec ladite chambre de dosage (9) et utilisable pour faire varier le volume de cela et monté pour déplacement vers l'extérieur par réaction à une accroissement en volume du fluide dans ladite chambre de dosage et pour déplacement à la main vers l'intérieur;
moyen (14) associé avec ledit système à piston secondaire (10) pour indiquer le volume de fluide dans ladite chambre de dosage (9);

lesdits systèmes à pistons primaire et secondaire (6, 10) étant coaxiaux, ledit système à piston primaire (6) ayant une extrémité intérieure et une extrémité extérieure, ledit système à piston secondaire (10) se prolongeant axialement au travers dudit système à piston primaire avec l'extrémité intérieure du système à piston secondaire dans ladite chambre de dosage (9) et l'extrémité extérieure du système à piston secondaire à l'extrémité extérieure dudit système à piston primaire;
moyens (4, 7) pour empêcher le déplacement relatif entre ledit système à piston primaire (6) et ledit corps (1) lorsque ledit système à piston secondaire (10) est déplacé pour éjecter le fluide de la chambre de dosage (9);
moyen d'étanchement (16) à montage adjacent à l'ajutage de passage dudit réservoir (17) et sélectivement ouvrable pour permettre le mouvement du fluide au travers de cela;
caractérisé en ce que lesdits moyens (4, 7) pour empêcher le mouvement relatif comprend un système de vis à filets (4, 7) en prise sur et entre ledit système à piston primaire (6) et ledit corps (1) disposés tels que le mouvement rotatoir dudit système à piston primaire (6) par rapport audit corps (1) provoquera un déplacement longitudinal dudit système à piston primaire (6) par rapport audit corps et le réservoir de contenance en cela;
ledit moyen pour indiquer le volume de fluide dans ladite chambre de dosage (9) comprend une poignée tubulaire (12) placée par-dessus ledit système à piston secondaire (10) et ayant làdessus les graduations (14);
moyens (18, 20) sur ladite poignée (12) pour limiter par ajustage le déplacement vers l'extérieur dudit système à piston secondaire (10); et
ladite chambre de dosage (9) est définie par un orifice de chemise allongée (9a) au travers de l'extrémité intérieure dudit système à piston primaire (6), ledit système à piston secondaire (10) étant introduit dans ladite chemise, ledit moyen étant pour limiter par ajustage le déplacement vers l'extérieur dudit système à piston secondaire (10) comprenant un écrou (20) monté et réglé sur ladite chemise (9a) et en prise pour son mouvement rotatoire par l'intérieur de ladite poignée tubulaire (12) étant longitudinalement coulissable par-dessus ledit écrou, et un moyen de butée (12a) sur ladite poignée pouvant être en prise avec l'écrou, selon le réglage, pour limiter le mouvement vers l'extérieur de ladite poignée (12) et dudit système à piston secondaire (10).

2. Une seringue selon Revendication 1, dans laquelle ledit moyen d'étanchement comprend une rondelle souple (16) pouvant être pénétrée par une aiguille hypodermique et qui se ferme une fois que l'aiguille en est extraite.

3. Une seringue selon Revendication 1 ou Revendication 2, dans lesquelles la graduation (8a) pour calibrage radial est prévue sur un collet circonférentiel (8) sur et ceinturant ledit système à piston primaire (6).

Fig. 1.

Fig. 2.